# EUROPEAN PATENT SPECIFICATION

(11) **EP 0 498 339 B1**
(45) Date of publication and mention of the grant of the patent: **08.11.1995**
(21) Application number: 92101719.0
(22) Date of filing: 03.02.1992
(51) Int. Cl.: B05D 1/18, C09D 4/00

(54) **Object comprising an ornament and thereon a monomolecular film**
Objekt mit einem Ornament und darauf einem monomolekularen Film
Objet avec un ornement portant un film monomoléculaire

(30) Priority: 06.02.1991 JP 38134/91; 30.04.1991 JP 98902/91
(43) Date of publication of application: 12.08.1992
(73) Proprietor: MATSUSHITA ELECTRIC INDUSTRIAL CO., LTD., Kadoma-shi, Osaka-fu, 571 (JP)
(72) Inventor: Ogawa, Kazufumi, Hirakata-shi 573, Osaka (JP); Mino, Norihisa, Settsu-shi 566, Osaka (JP); Soga, Mamoru, Osaka-shi 543 (JP)
(74) Representative: Grünecker, Kinkeldey, Stockmair & Schwanhäusser Anwaltssozietät

(56) References cited:
- WORLD PATENTS INDEX Week 50, Derwent Publications Ltd., London, GB; AN 77- 88959Y & JP-A-52 662 (SUWA SEIKOSHA K.K. & HAMASAWA KOGYO K.K.) 2 November 1977
- PATENT ABSTRACTS OF JAPAN vol. 14, no. 152 (M-953)23 March 1990 & JP-A-2 015 101 (MATSUSHITA ELECTRONIC IND.CO.) 18 January 1990

## Description

In various fields, there is a demand for improving the surface of materials such as plastics, metals, ceramics, fibers, wood, concrete and coatings to meet various purposes.

For example, it has been well known in the art to improve the surface of polymers. For example, a surface can be made water- and oil-repelling by coating it with a fluorine-containing silane coupling material. To make a surface hydrophilic, it can be coated with polyvinyl alcohol. To make a surface anti-contaminating it can be coated with a suspension of a fluorocarbon-based polymer.

However, conventionally obtained coating films are weakly bonded to polymer-containing materials or coated substrates and become separated by repeated wiping of the surface with a cloth or washing the surface with water. This results in a loss of the beneficial effects of the surface treatment. Moreover, the conventional coating films comprise randomly oriented molecules. Therefore, the films have many pin holes which deteriorate their characteristics. Further, fluorocarbon-based polymer coating films lack transparency and cannot be used on materials such as ornaments, and transparent plastic optical materials for which transparency is strongly demanded.

Ornaments, when contaminated, decrease in value and esteem. For ornaments, prevention of contaminants is most important. This applies to ornaments in actual use as well as to those displayed in department store show windows.

Heretofore, there has been no effective way to prevent contamination of ornaments.

Scrupulous wiping or washing of ornaments is very time-consuming, and certain kinds of contaminants cannot be readily removed without causing scars or scratches on the material.

JP-A- 2 015 101 discloses an overcoat for ultrafine particles consisting a chemically adsorbed monomolecular layer comprising a silane based surface active agent. The purpose of the overcoat is to maintain the properties of the ultrafine particles of a metal or a metal oxide while preventing them from spontaneous combustion in an atmosphere containing oxygen or water.

JP-A- 52 130 662 discloses a dial plate for a watch obtained by surface-treating a dial plate with polybutadiene and polymers derived therefrom to form on the dial plate surface an adsorbed monomolecular or multilayer film of polybutadiene. The obtained dial plate has an improved resistance to light, water and weather while the lustre, etc. of the dial plate are retained.

It is the object of the present invention to provide high performance ornaments which are highly water- and oil-repelling as well as anti-contaminating, i.e. contaminants will not attach thereto, or they can be readily removed therefrom if attached, and a method of forming such ornaments, specifically to provide on the surface thereof a highly durable, highly functional covalently bonded film.

According to the present invention this object is achieved with an object comprising an ornament and thereon a monomolecular film, characterized in that said film comprises a fluorocarbon group and is covalently bonded to the ornament surface and in that said ornament is selected from the group consisting of gem, pearl, watch, watch case, sapphire, ruby, emerald, garnet, cat's eye, diamond, topaz, quartz, crystal, agate, pottery, porcelain, ceramics, glass, stone, wood, and plastic.

Further, according to the present invention this object is achieved with a method of forming the above object comprising washing the ornament and dipping the ornament in a solution obtained by dissolving a chlorosilane-based surface active material having a chlorosilane group at one end and a fluorocarbon straight chain group at the other end in an organic solvent, thereby forming a covalently bonded monomolecular film of said surface active material on the entire surface of the ornament.

It is preferable in this invention that the fluorocarbon group containing monomolecular film is formed on the ornament substrate surface via a siloxane-based monomolecular film.

In this invention the ornament is selected from the group consisting of gem, pearl, watch, watch case, sapphire, ruby, emerald, garnet, cat's eye, diamond, topaz, quarz, crystal, agate, pottery, porcelain, ceramics, glass, stone, wood, and plastics.

It is preferable in this invention that the chlorosilane-based surface active matertial having a chlorosilane group at one end and a fluorocarbon straight chain group at the other end is represented by the general formula

CF₃-(CF₂)ₘ-R-SiXₚCl₃₋ₚ

wherein m represents 0 or an integer, R represents an alkyl group, an alkylene group or an alkyne group or a substituted group containing a -Si- group or an oxygen atom, X represents a hydrogen atom or an alkyl group or a substituted group containing a -Si- group or an oxygen atom, and p represents 0, 1 or 2.

In a preferred embodiment of the present invention the method of forming the object according to the invention comprises washing the ornament, contacting the ornament with a non-aqueous solution containing an inner layer material having a plurality of chlorosilyl groups, thereby causing a reaction between hydroxyl groups at the surface of the ornament and chlorosilyl groups of the inner layer material having a plurality of chlorosilyl groups to precipitate the material on the surface of the ornament, removing an excess of the material from the ornament surface using a non-aqueous solution, reacting the inner layer material with water, prior to said dipping the ornament in said solution obtained by dissolving said surface active material having a chlorosilane group at one end and a straight chain fluorocarbon group at the other end in an organic solvent, to form a laminated, covalently bonded monomolecular film on said ornament.

It is also preferable in this invention that the inner layer material containing a plurality of chlorosilyl groups is selected from SiCl₄, SiHCl₃, SiH₂Cl₂, and Cl(SiCl₂O)ₙCl₃ wherein n represents an integer from 1 to 20.

It is further preferable in the method of this invention that the chlorosilane-based surface active matertial having a chlorosilane group at one end and a fluorocarbon chain group at the other end is

CF₃-(CF₂)ₘ-R-SiXₚCl₃₋ₚ

where m represents 0 or an integer, R represents an alkyl group, an alkylene group or an alkyne group or a substituted group containing a -Si- group or an oxygen atom, X represents a hydrogen atom or an alkyl group or a substituted group containing a -Si- group or an oxygen atom, and p represents 0, 1 or 2.

According to this invention a film containing siloxane bonds may be covalently bonded to an ornament surface by contacting the ornament surface with a mixed solution containing at least a chlorosilane-based surface active material and a fluorine-containing non-aqueous solution without active hydrogen and which is capable of reacting with the chlorosilane-based surface active material.

It is preferable in this invention that the fluorine-containing non-aqueous solution contains a tertiary amine or cyclic ether.

It is further preferable in this invention that the ornament surface is treated with a member of the group consisting of dichlorosilane, trichlorosilane and tetrachlorosilane prior to forming the chemically adsorbed film.

It is preferable in this invention that the ornament is a polymer or has a polymer coating.

Figure 1 is a perspective view showing a pearl as a typical example of an ornament according to the invention.

Figure 2 is a perspective view showing a wrist watch as a typical ornament according to the invention.

Figure 3 is a schematic sectional view, enlarged to a molecular level, showing the surface of a pearl according to the invention.

Figure 4 is a schematic sectional view, enlarged to a molecular level, showing a wrist watch band surface embodying the invention.

Figure 5 is a schematic sectional view, enlarged to a molecular level, showing a wrist watch band surface during processing.

Figure 6 is a schematic sectional view, enlarged to a molecular level, showing a wrist watch band surface after processing.

Figure 7 is a sectional view showing a polymer composition obtainable by a method embodying the invention.

This invention relates to an object comprising an ornament having a thin film formed on the ornament surface. The thin film is a fluorocarbon-based monomolecular film which is covalently bonded to the ornament surface. By contacting the ornament with the non-aqueous solution containing a chlorosilane group at one end and a fluorocarbon chain at the other end, a reaction occurs between hydroxyl groups at the ornament surface and the chlorosilyl groups of the material having a plurality of chlorosilyl groups. A monomolecular film which is covalently bonded to the ornament surface is thereby obtained. A thin, fluorine-containing, monomolecular film thus can be formed on the ornament such that it is chemically bonded to the ornament. The film is anti-contaminating, water- and oil-repelling, durable and does not spoil the intrinsic luster of the ornament.

According to the invention, a covalently bonded film is formed on an ornament surface via siloxane bonds. It is thus possible to obtain a highly durable, highly functional covalently bonded film compared to those in the prior art. In addition, a fluorine-containing non-aqueous solution not containing any active hydrogen capable of reacting with a chlorosilane-based surface active material is used. Thus, it is possible to obtain a monomolecular film which is substantially pin-hole free and has a high density without any damage.

Further. according to a preferred embodiment of the invention, the fluorine-containing non-aqueous solution contains a tertiary amine or cyclic ether. Thus, the highly functional covalently bonded film may be formed without dissolving the substrate.

In a further preferred embodiment of the invention, prior to a reaction step to form the monomolecular film, the ornament surface is treated with a material having a plurality of chrolosilyl groups such as dichrolosilane, trichlorosilane or tetrachlorosilane. It is thus possible to provide siloxane bonds at a high density on the substrate surface.

In yet a further preferred embodiment of the invention, the ornament surface is a polymer coating. It is thus possible to form the highly functional monomolecular film according to the invention on a polymer coated surface which has been comparatively difficult in the prior art.

In the method according to the invention, the various ornaments as mentioned above can be used, for example plastics, ceramics, fibers, wood and concrete. A polymer material is preferred. The invention can be widely applied to the following polymer surfaces.

Examples of resin(s): a polyolefin such as a polypropylene and polyethylene, a polyvinylchloride plastic, a polyamide, a polyimide, a polyamideimide, a polyester, an aromatic polyester, a polycarbonate, a polystyrene, a polysulfide, a polysulfone, a polyethersulfone, a polyphenylenesulfide, a phenolic resin, a furan resin, a urea resin, an epoxy resin, a polyurethane, a silicon resin, an ABS resin, a methacrylic resin, an acrylate resin, a polyacetal, a polyphenylene oxide, a polymethylpentene, a melamine resin, an alkyd resin and an unsaturated polyester cured resin. Thus, any general-purpose plastic material may be used. Such plastic material may contain a filler, e.g., carbon dioxide, calcium carbonate and titanium oxide, or a plastisizer, e.g., dibutyl phthalate. Further, it may be colored with a dye or a pigment.

Where it is desired to form a polymer ornament that is water- and oil-repelling or anti-contaminating or slippery, an alkyl fluoride-containing chlorosilane surface active material is used as a material for forming the covalently bonded film. When it is desired to form a polymer composition according to the invention that is super-hydrophilic, after forming a chemically adsorbed film by using a chlorosilane-based surface active material which contains an alkyl group with an end vinyl group, the end is converted into a hydroxyl group by irradiating the film with, e.g., an electron beam, X-rays, gamma rays or ultraviolet rays in an oxygen atmosphere. For example, a chemically adsorbed film is formed by using tetrachlorosilane and then reacting with water to convert the chlorosilyl bonds to silanol bonds.

Examples of the chlorosilane-based surface active material having an alkyl fluoride group are such trichlorosilane-based surface active materials as
CF₃(CH₂)ᵣSiXₚCl₃₋ₚ,
CF₃(CF₂)ₛO(CH₂)ₜSiXₚCl₃₋ₚ,
CF₃(CF₂)ᵤSi(CH₃)₂(CH₂)ᵥSiXₚCl₃₋ₚ,
and CF₃COO(CH₂)_{w}SiXₚCl₃₋ₚ,
wherein r is from 1 to 25, s is from 0 to 12, t is from 1 to 20, u is from 0 to 12, v is from 1 to 20, w is from 1 to 25. These surface active materials are suitably represented by the following examples:
CF₃CH₂O(CH₂)₁₅SiCl₃,
CF₃(CF₂)₂Si(CH₃)₂(CH₂)₁₅SiCl₃,
CF₃(CH₂)₂Si(CH₃)₂(CH₂)₁₅SiCl₃,
CF₃(CF₂)₂Si(CH₃)₂(CH₂)₆SiCl₃,
CF₃(CF₂)₃(CH₂)₂Si(CH₃)₂(CH₂)₉SiCl₃,
CF₃COO(CH₂)₁₅SiCl₃,
CF₃(CF₂)₉(CH₂)₂SiCl₃,
CF₃(CF₂)₇(CH₂)₂SiCl₃, and
CF₃(CF₂)₅(CH₂)₂SiCl₃.

Particularly, trichlorosilane-based surface active materials are suitable because chlorosilyl bonds, other than those coupled to their hydrophilic groups, form inter-molecular bonds with adjacent chlorosilane groups and with siloxane bonds. This way a firmer film can be formed. Further, a trichlorosilane surface active material of the formula CF₃(CF₂)ₙ(CH₂)₂SiCl₃ wherein n represents an integer, most suitably 3 to 25, is preferred. This compound has a desirable solubility and chemical adsorption property. It is also water-repelling, anti-contaminating and has other desirable functional properties. Further, with an ethylene or acetylene group added to or incorporated in the alkyl fluoride chain portion, the monomolecular film may be crosslinked after formation by irradiating it with an electron beam of about 5 Mrads, thereby further improving the hardness of the film.

Examples of the chlorosilane-based surface active material containing an alkyl group are such trichlorosilane-based surface active materials as
CH₃(CH₂)ᵣSiXₚCl₃₋ₚ,
CH₃(CH₂)ₛO(CH₂)ₜSixₚCl₃₋ₚ,
CH₃(CH₂)ᵤSi(CH₃)₂(CH₂)ᵥSiXₚCl₃₋ₚ,
and CH₃COO(CH₂)_{w}SiXₚCl₃₋ₚ,
wherein r is from 1 to 25. s is from 0 to 12. t is from 1 to 20, u is from 0 to 12, v is from 1 to 20, w is from 1 to 25. These surface active materials are suitably represented by the following examples:
CH₃CH₂O(CH₂)₁₅SiCl₃,
CH₃(CF₂)₂Si(CH₃)₂(CH₂)₁₅SiCl₃,
CH₃(CH₂)₂Si(CH₃)₂(CH₂)₁₅SiCl₃,
CH₃(CH₂)₂Si(CH₃)₂(CH₂)₆SiCl₃,
CH₃(CH₂)₅Si(CH₃)₂(CH₂)₉SiCl₃,
CH₃COO(CH₂)₁₅SiCl₃,
CH₃(CH₂)₉(CH₂)₂SiCl₃,
CH₃(CH₂)₇(CH₂)₂SiCl₃, and
CH₃(CH₂)₅(CH₂)₂SiCl₃,
and such lower-alkyl substituted monochlorosilane- or dichlorosilane-based surface active materials. Particularly,
CH₃(CH₂)ₙSiCl₃
wherein n represents an integer, most suitably 3 to 25, is preferable because of its desirable solubility. Further, the alkyl group in the chlorosilane-based surface active material may contain an end vinyl group as in the formula

CH₂=CH-(CH₂)ₙSiCl₃

wherein n represents an integer, preferably 3 to 25.

The chlorosilane-based surface active material according to the invention is not limited to those in the form of a straight chain as noted above. It is possible to use those having a branched alkyl fluoride or hydrocarbon group or those in a form wherein the silicon at one end is substituted with an alkyl fluoride or a hydrocarbon group (i.e., those represented by a formula R₂SiCl₂, R₃SiCl, R¹R²SiCl₂ or R¹R²R³SiCl wherein R, R¹ , R² and R³ represents an aklyl fluoride or a hydrocarbon group). To increase the adsorption density, however, the straight chain form is preferred.

Further, by chemically asdorbing a material for forming an inner layer material having a plurality of chlorosilyl groups, e.g., SiCl₄, SiHCl₃, SiH₂Cl₂, and Cl(SiCl₂O)ₙCl₃ (wherein n represents an integer from 1 to 20), SiClₘ(CH₃)₄₋ₘ, SiClₘ(C₂H₅)₄₋ₘ (wherein m represents 1, 2 or 3), and HSiClₚ(CH₃)₃₋ₚ, HSiClₚ(C₂H₅)₃₋ₚ (wherein p represents 1 or 2), and then reacting it with water, surface chlorosilyl bonds are converted to hydrophilic silanol bonds, thereby making the polymer ornament hydrophilic. Among the materials containing a plurality of chlorosilyl groups, tetrachlorosilane (SiCl₄ ) is suitable in that it is highly reactive and low in molecular weight, thus providing silanol bonds at a high density. In this way, the polymer ornament may be made highly hydrophilic compared to the oxidation of a polymer-containing ornament or an ornament polymer coating material. The polymer is thus made super-hydrophilic and may be used in situ. A chlorosilane-based surface active material containing an alkyl fluoride group may be chemically adsorbed to the polymer. A film obtained in this way has a high density and has enhanced water- and oil-repelling and anti-contaminating properties.

The substrate according to the invention must contain on its surface active hydrogen groups such as -OH, -COOH, -NH₂ or =NH groups.

The polymer ornament is obtained according to the invention by a method which comprises the step of making a polymer-containing ornament surface hydrophilic by oxidization, and the step of forming a film containing siloxane bonds by covalently bonding a chlorosilane-based surface active material on the oxidized surface by dipping the surface in a fluorine-containing non-aqueous solution.

The polymer-containing ornament may be oxidized by the usual means, e.g., an oxygen plasma treatment, a corona treatment or a method of dipping the material in a blend solution containing concentrated sulfuric acid and potassium dichromate (i.e., a chromium blend liquid treatment).

As the fluorine-containing non-aqueous solution for the method of obtaining a polymer ornament according to the invention may be used any solution which does not permit dissolution of a polymer contained in the material with a monomolecular film to be covalently bonded thereto and which is free from active hydrogen capable of reacting with the chlorosilane-based surface active material. Suitable examples are tertiary amines containing a fluoroalkyl group or a cyclic ether having a fluoroalkyl group. Examples of the tertiary amines are compounds having the formula N(CₙF₂ₙ₊₁)₃ wherein n represents an integer from 2 to 10, the fluoroalkyl group being either straight or branched. Examples of the cyclic ether are those having the formula: 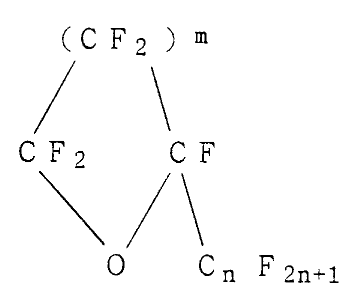
(wherein m represents an integer from 2 to 8, n represents an integer from 0 to 10)
As the covalently bonded film formed on the surface of a polymer ornament according to the invention, a single covalently bonded monomolecular layer is sufficient. To form only a single layer as a covalently bonded monomolecular film, it is only necessary to chemically adsorb the chlorosilane-based surface active material or a material containing a plurality of chlorosilyl groups and then wash away the non-aqueous solution without contact with moisture. No other specific process is needed. The covalently bonded film may of course be a laminated monomolecular film. Where the covalently bonded film includes a laminated layer, the functional groups are oriented to improve the density and provide for high functional performance.

A typical embodiment of the invention is an ornament having a thin film formed on its surface. The thin film includes at least a fluorine-containing chemically adsorbed monomolecular layer which is chemically bonded to the ornament material. It is thus possible to obtain a highly anti-contaminating, high performance ornament, in which contaminants will not attach, or from which they may be easily removed. Further, the ornament surface will have an improved smoothness with a high resistance to scars and scratches.

Further, according to the invention a fluorocarbon-based monomolecular film having a thickness in the nanometer range is formed on the ornament surface. Therefore, the luster intrinsic of the ornament is not spoiled. The film is a carbon fluoride-based monomolecular film and has excellent water- and oil-repelling properties, brilliant surface, and provides an enhanced anti-contaminating effect. A high performance ornament thus can be provided which is highly water- and oil-repelling and anti-contaminating particularly with respect to sweat and dirt.

Further, the method as described above according to the invention permits efficient formation of a thin film according to the invention.

Ceramic ornaments usually contain hydroxyl groups at their surface. Thus, a fluorocarbon-based lamination monomolecular film may be formed on the ornament surface by a method which comprises the step of contacting the ornament with a non-aqueous solution containing straight carbon chain molecules having a chlorosilane group (SiClₙX₃₋ₙ, wherein n represents 1, 2 or 3, X represents a functional group) at one end, e.g., a chlorosilane surface active material having a fluorocarbon group and a chlorosilane group for forming a monomolecular film on the ornament surface by a reaction occurring between hydroxyl groups at the ornament surface and chlorosilyl groups of the material, or contacting the ornament with a non-aqueous solution containing a material having a plurality of chlorosilyl groups to precipitate the material having a plurality of chlorosilyl groups on the ornament surface by a reaction occurring between hydroxyl groups at the ornament surface and chlorosilyl groups of the material, a step of washing away excess material having a plurality of chlorosilyl groups remaining on the ornament using a non-aqueous organic solvent followed by washing with water. Thus forming a siloxane-based monomolecular film of the material having a plurality of chlorosilyl groups on the ornament, and a step of forming a lamination monomolecular film by covalently bonding a silane-based surface active material having a straight carbon chain with a chlorosilane group at one end to the ornament.

In the following, examples of the invention will be given.

### Example 1

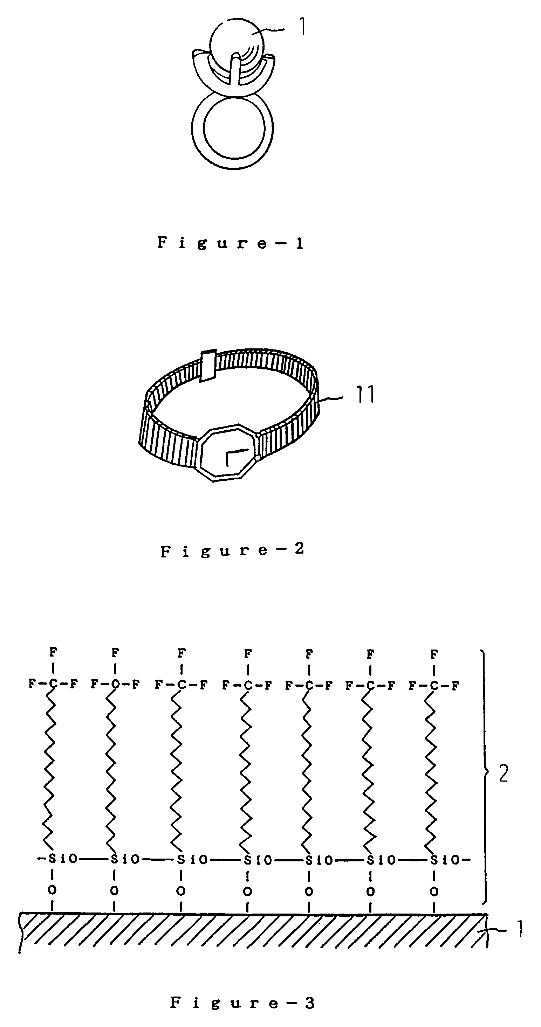

A natural pearl 1 (Figure 1) was dipped and held for 2 h in a non-aqueous solution containing a material having a fluorocarbon group and a chlorosilane group, e.g., "Fluorinert® FC-40" (products by 3M corp., a fluorine-based solution) which was obtained by dissolving 1 wt. % of
CF₃(CF₂)₇(CH₂)₂SiCl₃
Since the pearl is mainly composed of calcium carbonate and apachite, its surface contains numerous hydroxyl groups. Thus, a dehydrochlorination reaction was brought about between -SiCl groups of the material having a fluorocarbon group and a chlorsilane group and hydroxyl groups. This reaction is represented by the following formula [1].

CF₃(CF₂)₇(CH₂)₂-SiCl₃+(-OH)

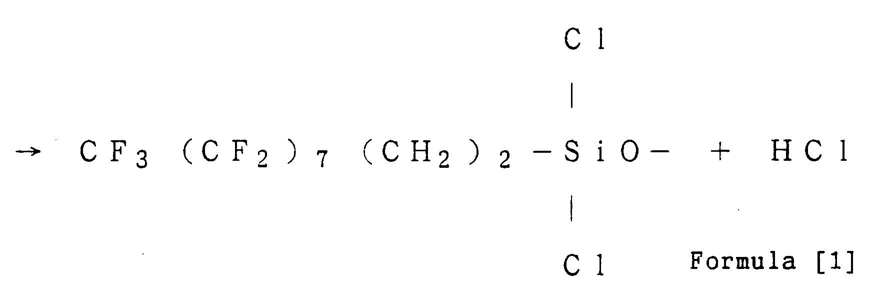

The pearl 1 was then washed with "Fluorinert® FC-40" to remove unreacted material remaining on the surface, followed by washing with water or exposure to air containing moisture. The -SiCl group was converted to a -SiOH group as in formula [2]. 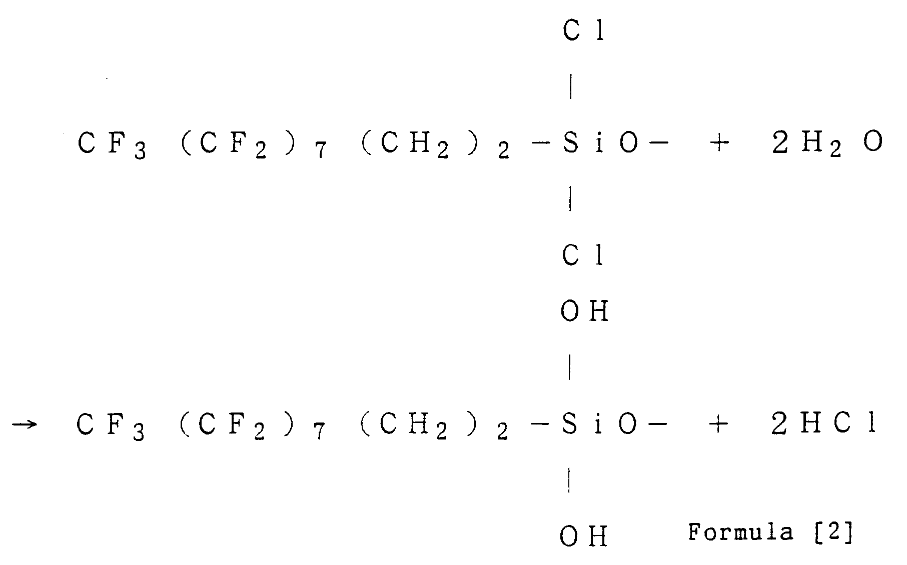
Each silanol group (-SiOH) was then dehydrated and crosslinked to form a siloxane bond (-SiO-) after drying as in formula [3]. The drying temperature may be room temperature or above. 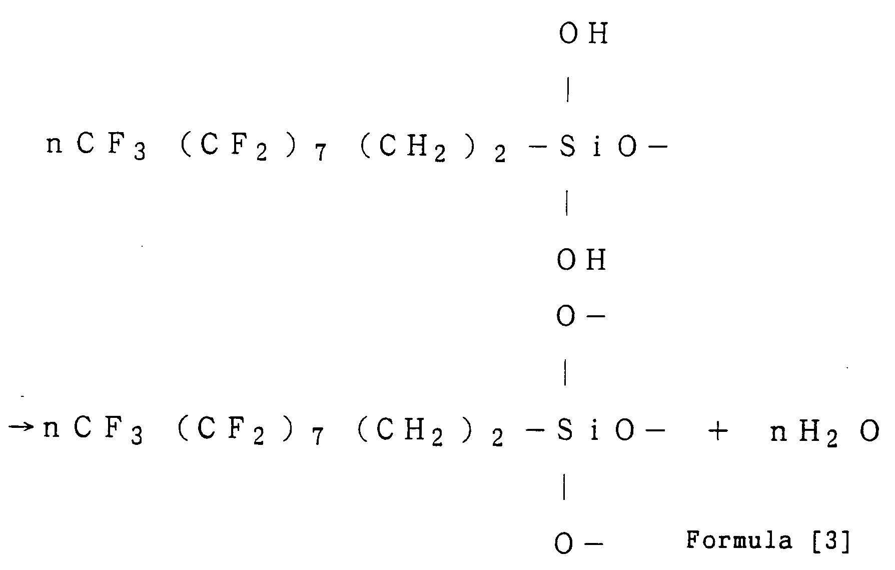
A covalently bonded monomolecular film 2 could be obtained on the surface of the pearl 1 as shown Figure 3. The monomolecular film 2 has a fluorine group and is chemically bonded (i.e., covalently bonded) to the pearl 1. The chemical bond is via a siloxane bond. The formation of the monomolecular film 2 was measured by FTIR spectrometry and the thickness was 1.5 nm. The film was firmly bonded to the pearl so that it did not separate.

Similar monomolecular films can be similarly formed on ornaments of, e.g., sapphire, ruby, emerald, garnet, cat's eye, diamond, topaz, quarz, crystal, agate, glass, and with the surface covered by an oxide film conaining hydroxyl groups. In this film formation, the adsorption time was adjusted appropriately. It had been thought that no natural oxide film was formed on the surface platinum and gold. Actually, however, the same effects as above can be obtained. This is presumably attributable to a very thin oxide film covering the surface.

The processed pearl was actually used to find that the attachment of contaminants can be greatly reduced compared to a non-processed pearl. Contaminants, if attached, can be easily removed by merely rubbing the surface with a brush with no scar or scratch being produced. Further, oily contaminants can be removed by merely washing with water. The luster of the pearl was not lost when contacted with sweat or dirt.

### Example 2

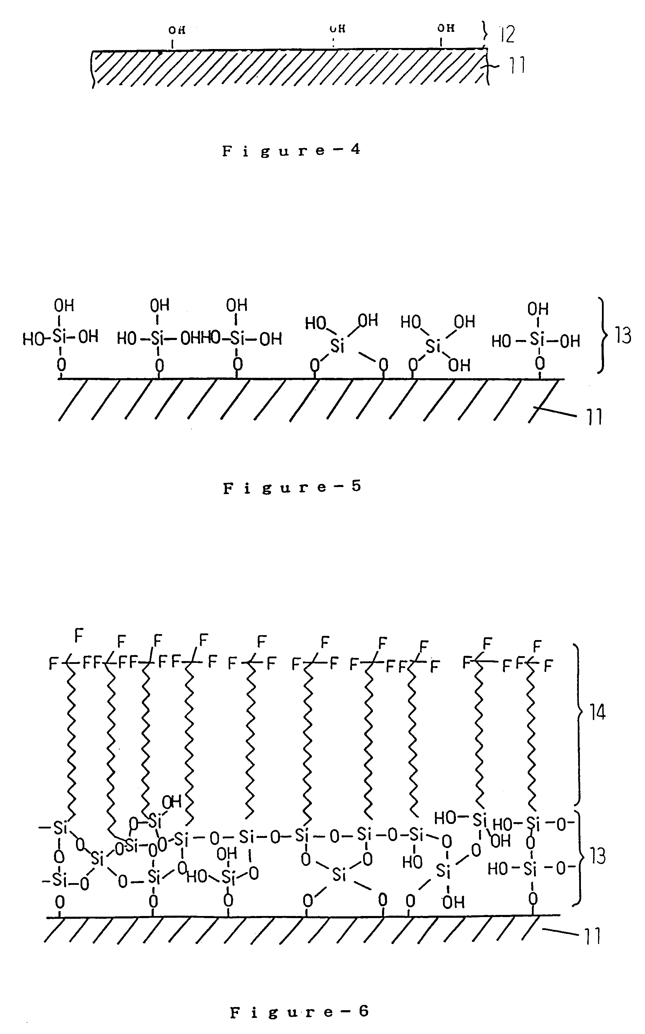

As a metal ornament, which was hydrophilic but contained fewer hydroxyl groups, a stainless steel wrist watch band 11 (Figure 2) was processed. The same effects are obtainable with glass frames made of metals such as titanium, aluminum, stainless steel and silver. The wrist watch band was dipped and held for 30 min in a solution prepared by dissolving 1 % by weight of a material having a plurality of trichlorosilyl groups, e.g., SiCl₄, SiHCl₃, SiH₂Cl₂, or Cl(SiCl₂O)ₙCl₃ (wherein n represents an integer in the range from 1 to 20) in a non-aqueous solvent, e. g., a chloroform solvent. SiCl₄ being particularly preferred since it is small in molecular size and active with respect to the hydroxyl groups, thus providing a wrist watch band surface which is uniformly hydrophilic. As a result, a hydrochloric acid removal reaction was brought about on the surface due to some -OH groups 12 present at the wrist watch band surface (Figure 4). A chlorosilane monomolecular film of the material having a plurality of trichlorosilyl groups was thus formed.

By using SiCl₄ as the material containing a plurality of trichlorosilyl groups, for example, molecules represented by formulas 4 and/or 5: 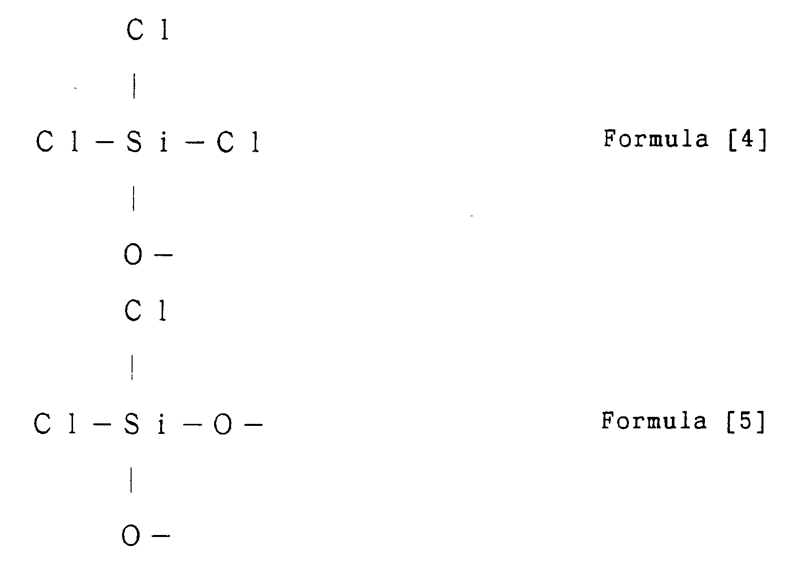
were secured via -SiO- bonds to the surface through a hydrochloric acid removal reaction brought about on the surface due to a small amount of hydrophilic -OH groups exposed at the surface of the wrist watch band 11.

By subsequently washing with a non-aqueous solvent, e.g., chloroform, and then with water, unreacted SiCl₄ molecules were removed. A siloxane monomolecular film 13 represented by formula 6 and/or 7 was obtained on the wrist watch band surface (Figure 5). 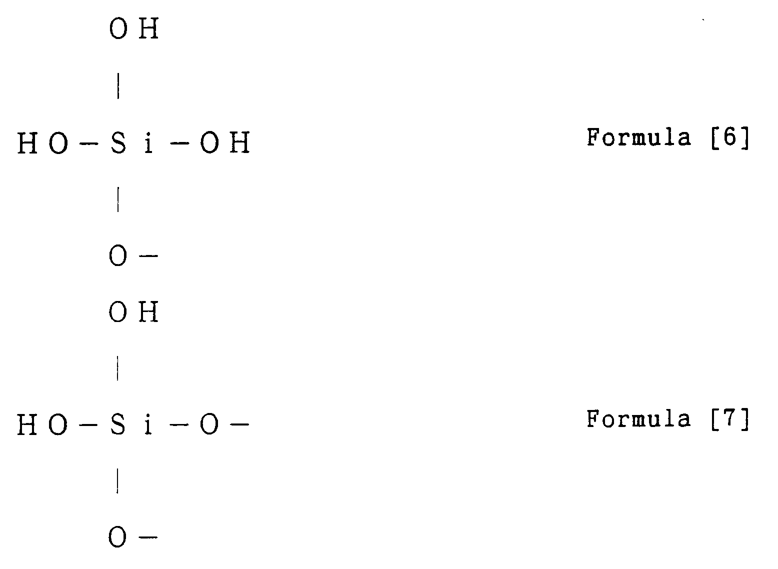
The monomolecular film 13 obtained in this way was bonded by chemical bonds of -SiO- to the ornament and did not separate. In addition, its surface contained numerous -SiOH bonds, corresponding in number to about three times the initial number of hydroxyl groups.

Further, the wrist watch band provided with the monomolecular film having numerous -SiOH bonds at the surface, was dipped and held for 1 h in an aqueous solution containing a material having a fluorocarbon group and a chlorosilane group, e.g., a solution obtained by dissolving 1 wt. % of
CF₃(CF₂)₇(CH₂)₂SiCl₃
in a solvent containing 80 of n-hexadecane, 12 % of carbon tetrachloride and 8 % of chloroform, thus producing bonds of
CF₃(CF₂)₇(CH₂)₂Si(O-)₃
on the wrist watch band surface. This reaction proceeded substantially the same as shown in formulae [1] to [3] above. A fluorine-containing monomolecular film 14 was formed. The film was chemically bonded to the inner siloxane monomolecular film 13 (Figure 6). It was formed over the entire wrist watch band surface and had a thickness of 1.5 nm. It did not separate in a peel-off test.

With an ethylene or acetylene group added to or incorporated in the carbon chain portion, the monomolecular film can be crosslinked after formation by irradiating it with an electron beam of about 5 Mrads, thus further improving hardness.

As has been shown in the above example, a carbon fluoride-based monomoecular film having a thickness at the nanometer level is formed on the ornament surface without spoiling the luster intrinsic to the ornament. This film has excellent water- and oil-repelling properties and makes the ornament surface anti-contaminating. It is thus possible to provide a high performance ornament which is highly anti-contaminating with respect to sweat and dirt. Moreover, this effect permits a great reduction of the number of washings.

### Example 3

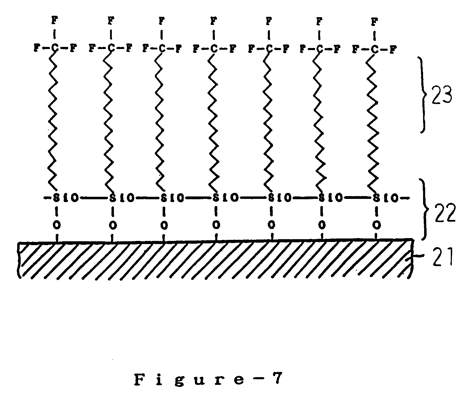

A polycarbonate substrate 21 (Figure 7) each side 5 cm long and 0.3 cm in thickness) was subjected to an oxygen plasma treatment in a commercially available UV dry stripper at an oxygen flow rate of 1 l/min to oxidize the surface. The substrate was then dipped and held in a tri(n-nonafluorobutyl)amine solution containing 10⁻² mol/l of heptadecafluorodecyltrichlorosilane used as the fluoroalkyl group-containing chlorosilyl-based surface active material in a nitrogen atmosphere at room temperature for 60 min. Unreacted heptadecafluorodecyltrichlorosilane was removed by washing with tri(n-nonafluorobutyl)amine solution and then washing with pure water. A fluoroalkyl group-containing covalently bonded monomolecular film 23 was formed on the polycarbonate substrate 21 via covalent siloxane bonds 22 (Figure 7). This reaction proceeded substantially as shown above in formulae [1] to [3].

### Example 4

The experiment was carried out like Example 3 except that an acrylic acid resin substrate was used in lieu of the polycarbonate substrate.

### Example 5

The experiment was carried out like Example 3 except that a polypropylene substrate was used in lieu of the polycarbonate substrate.

### Example 6

The experiment was carried out like Example 3 except that an ABS resin substrate was used in lieu of the polycarbonate substrate.

### Example 7

The experiment was carried out like Example 3 except that a poly(ethylene terephthalate) (PET) substrate was used in lieu of the polycarbonate substrate.

### Example 8

The experiment was carried out like Example 3 except that tri(nonafluoroisobutyl)amine was used in lieu of tri(n-nonafluorobutyl)amine.

### Example 9

The experiment was carried out like Example 3 except that tri(nonafluoroisobutyl)amine was used in lieu of tri(n-nonafluorobutyl)amine in Example 4.

### Example 10

The experiment was carried out like Example 3 except that tri(nonafluoroisobutyl)amine was used in lieu of tri(n-nonafluorobutyl)amine in Example 5.

### Example 11

The experiment was carried out like Example 3 except that tri(nonafluoroisobutyl)amine was used in lieu of tri-(n-nonafluorobutyl)amine in Example 6.

### Example 12

The experiment was carried out like Example 3 except that tri(nonafluoroisobutyl)amine was used in lieu of tri(n-nonafluorobutyl)amine in Example 7.

### Example 13

The experiment was carried out like Example 3 except that 2-(n-nonafluorobutyl)perfluorofuran was used in lieu of tri-(n-nonafluorobutyl)amine in Example 3.

### Example 14

The experiment was carried out like Example 3 except that 2-(n-nonafluorobutyl)perfluorofuran in lieu of (tri(n-nonafluorobutyl)amine in Example 4.

### Example 15

The experiment was carried out like Example 3 except that 2-(n-nonafluorobutyl)perfluorofuran was used in lieu of tri(n-nonafluorobutyl)amine in Example 5.

### Example 16

The experiment was carried out like Example 3 except that 2-(n-nonafluorobutyl)perfluorofuran was used in lieu of tri(n-nonafluorobutyl)amine in Example 6.

### Example 17

The experiment was carried out like Example 3 except that 2-(nonafluorobutyl)perfluorofuran was used in lieu of tri(n-nonafluorobutyl)amine in Example 7.

### Example 18

The experiment was carried out like Example 3 except that a butadiene-styrene rubber substrate was used in lieu of the polycarbonate substrate in Example 3.

### Example 19

The experiment was carried out like Example 3 except that a butyl rubber substrate was used in lieu of the polycarbonate substrate in Example 3.

### Example 20

The experiment was carried out like Example 3 except that a nitrile rubber substrate was used in lieu of the polycarbonate substrate in Example 3.

### Example 21

The experiment was carried out like Example 3 except that a method of dipping and holding the substrate for 5 min in concentrated sulfuric acid containing 10 % by weight of potassium dichromate was used in lieu of the method of oxidization.

### Comparative example 1

A silane coupling material (i.e., a methanol solution containing 2 % by weight of heptadecafluorodecyltrimethoxysilane) was spin coated on a polycarbonate substrate surface and then dried at 20 °C for 1 h.

### Comparative example 2

A covalently bonded monomolecular film of heptadecafluorodecyltrichlorosilane was formed without oxidization of the polycarbonate substrate in Example 3.

### Comparative example 3

A suspension of polytetrafluoroethylene was spray coated on the polycarbonate substrate surface in Example 3 and then thermally dried at 120 °C for 1 h.

The contact angles of the samples prepared in Examples 3 to 21 and Comparative examples 1 and 2 were examined. The contact angle was measured right after formation of the covalently bonded film or coating film and after rubbing the film surface 10,000 times with a water-wetted cloth. Table 1 shows the results.

**Table 1**

| | Contact angle ( ° ) with respect to water | | Contact angle ( ° ) with respect to salad oil | |
|---|---|---|---|---|
| | Initial value | After test | Initial value | After test |
| Example 3 | 121 | 119 | 101 | 99 |
| Example 4 | 131 | 128 | 112 | 109 |
| Example 5 | 116 | 114 | 95 | 93 |
| Example 6 | 134 | 131 | 114 | 112 |
| Example 7 | 125 | 123 | 106 | 104 |
| Example 8 | 119 | 116 | 99 | 97 |
| Example 9 | 129 | 127 | 110 | 110 |
| Example 10 | 114 | 111 | 104 | 101 |
| Example 11 | 132 | 130 | 112 | 100 |
| Example 12 | 123 | 120 | 104 | 102 |
| Example 13 | 100 | 98 | 81 | 80 |
| Example 14 | 124 | 122 | 105 | 102 |
| Example 15 | 102 | 100 | 82 | 80 |
| Example 16 | 100 | 99 | 80 | 79 |
| Example 17 | 111 | 108 | 90 | 89 |
| Example 18 | 106 | 104 | 86 | 84 |
| Example 19 | 117 | 114 | 97 | 96 |
| Example 20 | 116 | 113 | 96 | 93 |
| Example 21 | 117 | 115 | 98 | 96 |
| Com. Ex. 1 | 93 | 45 | 61 | 12 |
| Com. Ex. 2 | 45 | 45 | 12 | 12 |

As observed from Table 1, the products obtained by the method according to the invention remained water- and oil-repelling or hydrophilic even after the surface had been rubbed repeatedly with water-wetted cloth. The sample of Comparative example 1, however, was no longer water- and oil-repelling. With the sample of Comparative example 2, wherein a polymer substrate was used without surface oxidization, it was impossible to form a covalently bonded film containing siloxane bonds.

The polymer substrates with the chemically adsorbed monomolecular film containing a fluoroalkyl group formed on the surface had an excellent anti-contaminating property. After the friction test, the sample of Example 3 was dipped in salad oil. Its surface was then wiped with tissue paper. Oil could be easily wiped away. The sample in Comparative example 1 had a surface oil film and was sticky even after its surface had been wiped several times with tissue paper.

The polymer substrate according to the invention can also be utilized with optical materials. The light transmisson factor of the polycarbonate substrate in Example 3 with respect to visible light was 92 %. With the sample of Comparative example 3, obtained by coating with polytetrafluoroethylene, the light transmission factor was reduced to 50 % or below, and the transparency was considered inferior approximating that of stained glass.

The covalently bonded film mentioned above in each of the examples was a single monomolecular film. However, the same functions could be obtained on a polymer substrate with a laminated covalently bonded monomolecular film or with a covalently bonded film formed without washing away unreacted chlorosilane-based surface active material. Further, in the above examples a relatively pure polymer substrate was used as the polymer-containing ornament, but the same results could be obtained with polymers incorporating, e.g., fillers, plastisizers or coloring agents.

As shown above, according to the invention a polymer-containing ornament surface is subjected to an oxidization treatment to form hydroxyl or like hydrophilic groups. A film containing siloxane bonds is formed on the substrate surface by covalently bonding a chlorosilane-based surface active material. Thus, a covalently bonded film can be readily formed without having any hydroxyl or like hydrophilic group as a repetition unit. In addition, a very high density covalently bonded film may be formed by merely diping a polymer-containing or polymer coated ornament in a fluorine-containing non-aqueous solution containing a chlorosilane-based surface active material. Thus, the covalently bonded film can be highly water- and oil-repelling, anti-contaminating or super-hydrophobic depending on the function of the chlorosilane-based surface active material used.

As has been shown, since the polymer ornament according to the invention is provided with a film containing siloxane bonds covalently bonded to the surface, excellent durability with respect to repeated washing can be obtained compared to that in the prior art. Since the film contains a fluorocarbon group, it is highly water- and oil-repelling and anti-contaminating.

Further, with the method of obtaining a polymer ornament according to the invention, by preliminarily oxidizing the polymer substrate surface, a film containing siloxane bonds can be covalently bonded by covalently bonding a chlorosilane-based surface active material to the substrate surface.

As has been shown, the invention is greatly beneficial to industry.

## Claims

1. Object comprising an ornament and thereon a monomolecular film, characterized in that said film comprises a fluorocarbon group and is covalently bonded to the ornament surface and in that said ornament is selected from the group consisting of gem, pearl, watch, watch case, sapphire, ruby, emerald, garnet, cat's eye, diamond, topaz, quartz, crystal, agate, pottery, porcelain, ceramics, glass, stone, wood, and plastic.

2. The object according to claim 1, wherein said fluorocarbon group containing monomolecular film is formed on said ornament substrate surface via a siloxane-based monomolecular film.

3. A method of forming the object according to claim 1, comprising washing the ornament and dipping the ornament in a solution obtained by dissolving a chlorosilane-based surface active material having a chlorosilane group at one end and a fluorocarbon straight chain group at the other end in an organic solvent, thereby forming a covalently bonded monomolecular film of said surface active material on the entire surface of the ornament.

4. The method according to claim 3, wherein said chlorosilane-based surface active material having a chlorosilane group at one end and a fluorocarbon straight chain group at the other end is represented by the general formula
CF₃-(CF₂)ₘ-R-SiXₚCl₃₋ₚ
wherein m represents 0 or an integer, R represents an alkyl group, an alkylene group or an alkyne group or a substituted group containing a -Si- group or an oxygen atom, X represents a hydrogen atom or an alkyl group or a substituted group containing a -Si- group or an oxygen atom, and p represents 0, 1 or 2.

5. The method according to claim 3 or 4 further comprising washing the ornament, contacting the ornament with a non-aqueous solution containing an inner layer material having a plurality of chlorosilyl groups, thereby causing a reaction between hydroxyl groups at the surface of the ornament and chlorosilyl groups of said inner layer material having a plurality of chlorosilyl groups to precipitate said material on the surface of the ornament, removing an excess of said material from the ornament surface using a non-aqueous solution and reacting said inner layer material with water, prior to said dipping the ornament in said solution obtained by dissolving said surface active material having a chlorosilane group at one end and a straight chain fluorocarbon at the other end in an organic solvent, to form a laminated, covalently bonded monomolecular film on said ornament.

6. The method according to claim 5, wherein said inner layer material containing a plurality of chlorosilyl groups is selected from SiCl₄, SiHCl₃, SiH₂Cl₂, and Cl(SiCl₂O)ₙCl₃, wherein n represents an integer from 1 to 20.

7. The method of claim 3, wherein said solution obtained by dissolving a chlorosilane-based surface active material in an organic solvent is a non-aqueous solution.

8. The method of claim 7, wherein said non-aqueous solution further contains a tertiary amine or cyclic ether.

9. The method of claim 5, wherein said inner layer material having a plurality of chlorosilyl groups is selected from dichlorosilane, trichlorosilane and tetrachlorosilane.

10. The method of claim 3, wherein said ornament is a polymer or has a polymer coating.

## Patentansprüche

1. Gegenstand, umfassend ein Ornament und darauf einen monomolekularen Film, gekennzeichnet dadurch, daß der Film eine Fluorkohlenstoffgruppe umfaßt und kovalent an die Ornamentenoberfläche gebunden ist, und dadurch, daß das Ornament aus der Gruppe, die aus Edelstein, Perle, Uhr, Uhrengehäuse, Saphir, Rubin, Smaragd, Granat, Katzenauge, Diamant, Topas, Quarz, Kristall, Achat, Töpferwaren, Porzellan, Keramiken, Glas, Stein, Holz und Kunststoff besteht, ausgewählt wird.

2. Der Gegenstand nach Anspruch 1, worin der die Fluorkohlenstoffgruppe enthaltende, monomolekulare Film auf der Ornamentensubstratoberfläche über einen auf Siloxanbasierenden, monomolekularen Film ausgebildet wird.

3. Ein Verfahren zum Erzeugen des Gegenstandes nach Anspruch 1, das waschen des Ornamentes und Eintauchen des Ornamentes in eine Lösung umfasst, erhalten durch Auflösen eines auf Chlorsilan basierenden, oberflächenaktiven Materials, welches eine Chlorsilangruppe an einem Ende und eine geradkettige Fluorkohlenstoffgruppe an dem anderen Ende besitzt, in einem organischen Lösungsmittel, wodurch man einen kovalent gebundenen, monomolekularen Film des oberflächenaktiven Materials auf der ganzen Oberfläche des Ornamentes bildet.

4. Das Verfahren nach Anspruch 3, worin das auf Chlorsilan basierende, oberflächenaktive Material, welches eine Chlorsilangruppe an einem Ende und eine geradkettige Fluorkohlenstoffgruppe am anderen Ende besitzt, dargestellt wird durch die allgemeine Formel
CF₃-(CF₂)ₘ-R-SiXₚCl₃₋ₚ
worin m für 0 oder eine ganze Zahl steht, R eine Alkylgruppe, eine Alkengruppe oder eine Alkingruppe oder eine substituierte Gruppe mit einer -Si-Gruppe oder einem Sauerstoffatom darstellt, X ein Wasserstoffatom oder eine Alkylgruppe oder eine substituierte Gruppe mit einer -Si-Gruppe oder einem Sauerstoffatom darstellt, und P 0, 1 oder 2 darstellt.

5. Das Verfahren nach Anspruch 3 oder 4, das weiter umfasst Waschen des Ornamentes, in Kontakt bringen des Ornamentes mit einer nichtwäßrigen Lösung, die ein inneres Schichtmaterial mit einer Vielzahl von Chlorsilylgruppen enthält, wodurch man eine Reaktion zwischen Hydroxylgruppen auf der Oberfläche des Ornamentes und Chlorsilylgruppen des inneren Schichtmaterials, das eine Vielzahl von Chlorsilylgruppen enthält, bewirkt, um das Material auf der Oberfläche des Ornamentes abzuscheiden, Entfernen eines Überschusses des Materials von der Ornamentoberfläche, unter Verwendung einer nichtwäßrigen Lösung und Umsetzen des inneren Schichtmaterials mit Wasser, vor dem Eintauchen des Ornamentes in die Lösung, erhalten durch Auflösen des oberflächenaktiven Materials, welches eine Chlorsilangruppe an einem Ende und eine gerade Fluorkohlenstoffkette an dem anderen Ende besitzt, in einem organischen Lösungsmittel, um einen laminierten, kovalent gebundenen, monomolekularen Film auf dem Ornament zu bilden.

6. Das Verfahren nach Anspruch 5, worin das innere Schichtmaterial, das eine Vielzahl von Chlorsilylgruppen enthält, ausgewählt wird aus SiCl₄, SiHCl₃, SiH₂Cl₂, und Cl(SiCl₂O)ₙCl₃, worin n eine ganze Zahl von 1 bis 20 darstellt.

7. Das Verfahren nach Anspruch 3, worin die Lösung, erhalten durch Auflösen eines auf Chlorsilan basierenden, oberflächenaktiven Materials in einem organischen Lösungsmittel, eine nichtwäßrige Losung ist.

8. Das Verfahren nach Anspruch 7, worin die nichtwäßrige Lösung weiterhin ein tertiäres Amin oder einen zyklischen Ether enthält.

9. Das Verfahren nach Anspruch 5, worin das innere Schichtmaterial, welches eine Vielzahl von Chlorsilylgruppen besitzt, aus Dichlorsilan, Trichlorsilan und Tetrachlorsilan ausgewählt wird.

10. Das Verfahren nach Anspruch 3, worin das Ornament ein Polymer ist oder eine polymere Beschichtung hat.

## Revendications

1. Objet comprenant un ornement et, sur celui-ci, une pellicule monomoléculaire, caractérisé en ce que ladite pellicule comprend un groupe fluorocarboné et est liée de façon covalente à la surface de l'ornement et en ce que ledit ornement est choisi dans le groupe constitué d'une pierre précieuse, d'une perle, d'une montre, d'un boîtier de montre, d'un saphir, d'un rubis, d'une émeraude, d'un grenat, d'un oeil de chat, d'un diamant, d'une topaze, d'un quartz, d'un cristal, d'une agate, d'une poterie, d'une porcelaine, d'une céramique, d'un verre, d'une pierre, d'un bois et d'une matière plastique.

2. Objet selon la revendication 1, dans lequel ladite pellicule monomoléculaire contenant un groupe fluorocarboné est formée sur la surface dudit ornement substrat par l'intermédiaire d'une pellicule monomoléculaire à base de siloxane.

3. Procédé de formation de l'objet selon la revendication 1, comprenant le lavage de l'ornement et le trempage de l'ornement dans une solution obtenue en dissolvant une matière tensioactive à base de chlorosilane, ayant un groupe chlorosilane à une extrémité et un groupe fluorocarboné à chaîne linéaire à l'autre extrémité, dans un solvant organique, formant ainsi une pellicule monomoléculaire liée de façon covalente de ladite matière tensioactive sur la surface entière de l'ornement.

4. Procédé selon la revendication 3, dans lequel ladite matière tensioactive à base de chlorosilane ayant un groupe chlorosilane à une extrémité et un groupe fluorocarboné à chaîne linéaire à l'autre extrémité est représentée par la formule générale
CF₃-(CF₂)ₘ-R-SiXₚCl₃₋ₚ
où m représente 0 ou un nombre entier, R représente un groupe alkyle, un groupe alkylène ou un groupe alcyne ou un groupe substitué contenant un groupe -Si- ou un atome d'oxygène, X représente un atome d'hydrogène ou un groupe alkyle ou un groupe substitué contenant un groupe -Si- ou un atome d'oxygène, et p représente 0, 1 ou 2.

5. Procédé selon la revendication 3 ou 4, comprenant en outre le lavage de l'ornement, la mise en contact de l'ornement avec une solution non aqueuse contenant une matière de couche interne ayant plusieurs groupes chlorosilyles, provoquant ainsi une réaction entre les groupes hydroxyles à la surface de l'ornement et les groupes chlorosilyles de ladite matière de couche interne ayant plusieurs groupes chlorosilyles, pour précipiter ladite matière sur la surface de l'ornement, l'élimination d'un excès de ladite matière de la surface de l'ornement en utilisant une solution non aqueuse et la réaction de ladite matière de couche interne avec de l'eau, avant ledit trempage de l'ornement dans ladite solution obtenue en dissolvant ladite matière tensioactive ayant un groupe chlorosilane à une extrémité et un groupe fluorocarboné à chaîne linéaire à l'autre extrémité dans un solvant organique, pour former une pellicule monomoléculaire stratifiée liée de façon covalente sur ledit ornement.

6. Procédé selon la revendication 5, dans lequel ladite matière de couche interne contenant plusieurs groupes chlorosilyles est choisie parmi SiCl₄, SiHCl₃, SiH₂Cl₂ et Cl(SiCl₂O)ₙCl₃, où n représente un nombre entier de 1 à 20.

7. Procédé selon la revendication 3, dans lequel ladite solution obtenue en dissolvant une matière tensioactive à base de chlorosilane dans un solvant organique est une solution non aqueuse.

8. Procédé selon la revendication 7, dans lequel ladite solution non aqueuse contient en outre une amine tertiaire ou un éther cyclique.

9. Procédé selon la revendication 5, dans lequel ladite matière de couche interne ayant plusieurs groupes chlorosilyles est choisie parmi un dichlorosilane, un trichlorosilane et un tétrachlorosilane.

10. Procédé selon la revendication 3, dans lequel ledit ornement est un polymère ou a un revêtement polymère.
